# EUROPEAN PATENT SPECIFICATION

(11) **EP 0 781 264 B1**
(45) Date of publication and mention of the grant of the patent: **20.03.2002**
(21) Application number: 95936195.7
(22) Date of filing: 12.09.1995
(51) Int. Cl.: B01D 9/02, B01D 61/14, C07C 59/265, C07C 51/42, C07C 51/43

(54) **PROCESS FOR RECOVERING CITRIC ACID**
VERFAHREN ZUR WIEDERGEWINNUNG VON ZITRONENSÄURE
PROCEDE POUR RECUPERER L'ACIDE CITRIQUE

(30) Priority: 12.09.1994 ZA 9407011
(43) Date of publication of application: 02.07.1997
(73) Proprietor: AECI LIMITED, Johannesburg 2000 Transvaal (ZA); USTECH INC., Decatur, IL 62526 (US)
(72) Inventor: VERHOFF, Francis, H., Decatur, IL 62526 (US); GROND, Sanet, Centurion 0157 (ZA); HENDRICKS, Fadl, Sandton 2146 (ZA); RAMAN, Lakshminarayanan, Pattabiraman, Brooklyn Park, MN 55439 (US)
(74) Representative: Tiedtke, Harro, Dipl.-Ing.
(86) International application number: US9512160
(87) International publication number: WO9608459

(56) References cited:
- EP-A- 0 151 470
- EP-A- 0 479 084
- DE-C- 680 595
- GB-A- 1 494 414

## Description

This invention relates to a process for recovering citric acid.

Several separation techniques are known for separating low molecular weight components. Some of them are size-based and others are charge-based.

The former method is exemplified by nanofiltration. An application example thereof is described in EP-A-0 479 084. According to this method, granular citric acid and salts thereof are produced. Accordingly, a fermentation broth containing citric acid may be purified by ultrafiltration using a membrane that removes all solutes having a molecular weight greater than about 1.000 Dalton. Subsequent nanofiltration removes solutes with molecular weights above about 250 Daltons.

One application example of a charge-based separation method is described in GB-A-1,494,414. Accordingly, hyperfiltration is used for separating citric acid in aqueous solutions. In contrast to size-based separation such as ultrafiltration or nanofiltration, hyperfiltration is based on the ionic properties of the separation membrane. According to the mentioned document, a citric acid solution can pass through an acidified filtration membrane at a pH in the range of 5.5 to 9.7. On the other hand, citric acid can be held back on a membrane comprising basic groups.

However, the problem of separating citric acid from a less desirable component having a molecular weight very close to that of citric acid by nanofiltration remains still unsolved.

According to the invention, there is provided a process for purifying citric acid, which process comprises: (a) subjecting a liquid containing, in solution, citric acid as well as glucose and/or fructose having a similar molecular weight to citric acid, to nanofiltration in a filtration step; and (b) obtaining, from the filtration step, a permeate in which the ratio of the concentration of the citric acid to that of glucose and/or fructose is greater than the ratio of the concentration of the citric acid to that of glucose and/or fructose in the solution, wherein the rejection rate of citric acid on the nanofiltration membrane is lower than the rejection rate of glucose and/or fructose and the permeate is concentrated by subjecting it to crystallization without treating the permeate to remove the remaining glucose and/or fructose prior to the crystallization.

In other words, there is a greater degree of rejection of the less desirable component than of the citric acid in the filtration step. The nanofiltration will normally involve contacting the liquid with a nanofiltration membrane. Nanofiltration will naturally also separate the citric acid from any component with a molecular weight which is significantly greater than that of citric acid.

The Applicant believes that the process will have particular, but not necessarily exclusive, application in the treatment of fermentation broth to separate citric acid present therein as a fermentation product from residual glucose and/or fructose, thereby recovering the citric acid. It has been found that, with the process of the invention, the citric acid can be separated from residual glucose and/or fructose which can be undesirable. In other words, the process has specific application in the recovery of citric acid from a fermentation broth, particularly from a clarified citric acid fermentation broth.

The clarified citric acid fermentation broth can typically be that obtained by fermenting a carbohydrate feedstock to produce citric acid-rich fermentation broth and waste solids, and separating the broth from the solids.

Citric acid has a similar molecular mass to glucose and fructose and can preferentially be separated from glucose and/or fructose in the process according to the invention, as a result of its greater permeability through the nanofiltration membrane as compared to that of glucose and/or fructose.

The filtration step may be carried out at a concentration of the citric acid in the broth of 5%-30% by mass, preferably 10%-20% by mass, and the nanofiltration may be carried out at a temperature of 10°C-100°C, preferably 20°C-50°C. The pressure drop across the nanofiltration membrane will depend on the nature of the membrane and one the nature of the citric acid and the less desirable component to be separated and can be established by routine experimentation.

The clarified citric acid fermentation broth may, before the filtration step, be subjected to cation exchange to remove cations, such as potassium and magnesium ions, therefrom.

The process may include further treating the citric acid solution from the filtration step to purify it and/or to obtain a more concentrated citric acid fraction, or solid citric acid or a derivative of citric acid, such as sodium citrate.

Thus, the citric acid solution from the filtration step may be purified by anion exchange, e.g., to remove traces of anionic impurities, and/or by contacting it with activated carbon to remove traces of organic matter.

The purified citric acid solution may then be concentrated. This may include treating the solution to obtain solid pure citric acid and residual mother liquor. The concentration may include subjecting the solution to at least one crystallization sequence, e.g. by evaporation. In particular, the concentration may include passing the solution sequentially through an evaporator; a first crystallizer; a first centrifuge; optionally a dissolution tank, a second crystallizer and a second centrifuge; and producing mother liquor in the first centrifuge and, when present, in the second centrifuge. A portion of the mother liquor from the second centrifuge, when present, may then be recycled to the first crystallizer, while the mother liquor from the first centrifuge is withdrawn. The contacting of the citric acid solution with the activated carbon hereinbefore referred to may instead, or additionally, be effected after the purified citric acid solution has been concentrated at least partially, e.g., after it has passed through the evaporator.

The process may also include: (i) recycling a portion of the mother liquor from the first centrifuge to upstream of the evaporator; and/or (ii) withdrawing at least a portion of the mother liquor from the first centrifuge as a liquid product; and/or (iii) drying and/or granulating at least a portion of the mother liquor from the first centrifuge to obtain a solid citric acid/carbohydrate product; and/or (iv) treating at least a portion of the mother liquor from the first centrifuge, in a recovery step, to recover citric acid for recycle, or citrate salts as product.

When the process includes treating at least a portion of the mother liquid from the first centrifuge in a recovery step to recover citric acid, this citric acid may be recycled to upstream and/or downstream of the nanofiltration step. The treatment in the recovery step may then comprise one of the following: calcium citrate precipitation by adding lime thereto and redissolving with sulphuric acid; solvent extraction of citric acid utilizing a suitable solvent, followed by re-extraction of citric acid from the solvent into water using concentration differences or heating; ion exchange using a resin which selectively adsorbs citric acid, followed by elution; or various types of chromatography.

At least a portion of the retentate from the filtration step may be withdrawn as a liquid product. Instead, or additionally, at least a portion of the retentate from the filtration step may be dried or granulated to obtain a solid citric acid product. Instead, or additionally, at least a portion of the retentate from the filtration step may be treated in a citric acid recovery step, which may then be the same as the citric acid recovery step hereinbefore described, to recover citric acid or a derivative thereof therefrom.

The retentate from the filtration step may be combined with the mother liquor from the first centrifuge for withdrawal as a liquid product and/or for drying or granulating and/or for treatment in a recovery step, as hereinbefore described.

According to a preferred embodiment of the invention, the above-described process for recovering citric acid includes subjecting a clarified citric acid fermentation broth to nanofiltration in a filtration step to obtain, as a permeate, a purified citric acid solution.

The clarified citric acid fermentation broth may, before the filtration step, be subjected to cation exchange as hereinbefore described. The citric acid solution from the filtration step may be treated further to purify it and/or to obtain a more concentrated citric acid fraction, or solid citric acid or a derivative of citric acid, as hereinbefore described. The filtration step may also be as hereinbefore described.

The invention will now be described by way of example, with reference to the accompanying simplified flow diagram of a process according to the invention for treating a fermentation broth, and with reference to the non-limiting examples.

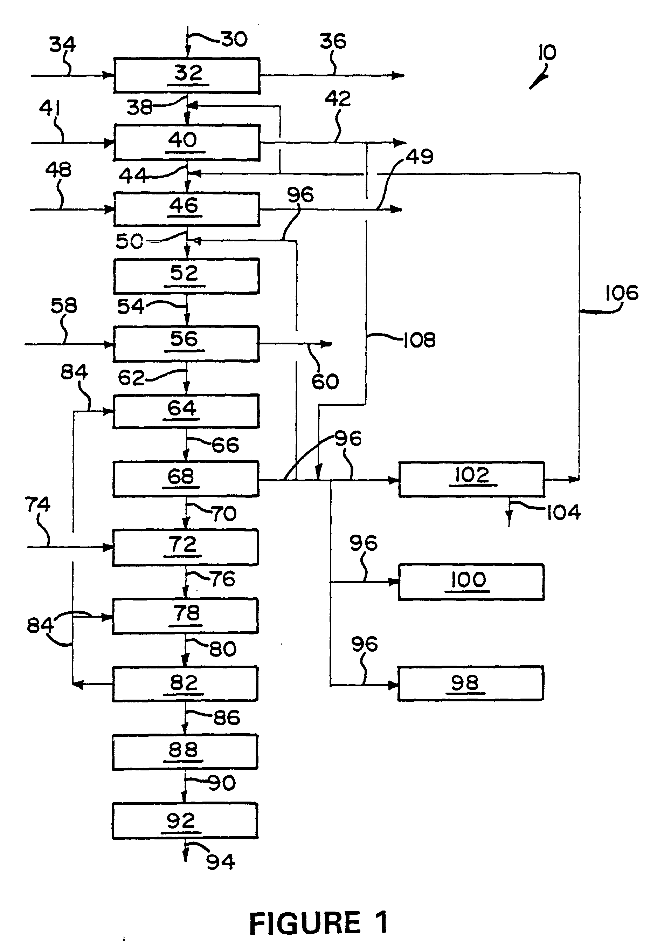

In the drawing, reference numeral 10 generally indicates a process according to the invention for treating a fermentation broth.

The process 10 includes a cation exchanger stage 32. A clarified citric acid fermentation broth feed line 30 leads from a fermentation stage (not shown) into the stage 32. A regeneration water/acid flow line 34 also leads into the stage 32, while a waste product withdrawal line 36 leads from the stage 32. A flow line 38 also leads from the stage 32.

The flow line 38 leads to a nanofiltration step or stage 40, with a waste product or retentate withdrawal line 42 leading from the stage 40. A cleaning water/base and diafiltration flow line 41 leads into the nanofiltration stage 40. A filtrate flow line 44 leads from the stage 40 to an anion exchanger 46, with a regeneration water/base flow line 48 leading into the exchanger 46. A waste product withdrawal line 49 leads from the stage 46, while a flow line 50 leads from the exchanger 46 to an activated carbon bed stage 52.

A flow line 54 leads from the stage 52 to an evaporation stage 56, with a steam flow line 58 leading into the stage 56. A condensate line 60 leads from the stage 56. A flow line 62 leads from the stage 56 to a first crystallization stage 64. A flow line 66 leads from the crystallization stage 64 to a first centrifugation stage 68. A flow line 70 leads from the first centrifugation stage 68 to a dissolution tank 72, with a water make-up line 74 leading into the tank 72. A flow line 76 leads from the tank 72 to a second crystallization stage 78, with a flow line 80 leading from the second crystallization stage 78 to a second centrifugation stage 82. A mother liquor recycle line 84 leads from the stage 82 to the crystallization stages 64, 78. A flow line 86 leads from the second centrifugation stage 82 to a drier 88, with a flow line 90 leading from the drier 88 to a screening stage 92. A solid product withdrawal line 94 leads from the screening stage 92.

The second crystallization stage 78 and second centrifugation stage 82 are used to improve crystal quality and are optional; they can be dispensed with, if necessary.

A mother liquor withdrawal line 96 leads from the first centrifugation stage 68.

In a first embodiment of the invention, the line 96 can be routed back to the flow line 50 for recycling a portion of the mother liquor.

In a second embodiment of the invention, the flow line 96 can lead to a suitable liquid product withdrawal stage 98.

In a third embodiment of the invention, the flow line 96 can lead to a drying and granulation stage 100.

In a fourth embodiment of the invention, the flow line 96 can lead to a recovery stage 102. A waste product withdrawal line 104 leads from the stage 102. A citric acid recycle line 106 leads from the stage 102 back to upstream and/or downstream of stage 40.

It will be appreciated that the first, second, third and fourth embodiments described hereinbefore are optional and can be used individually, or a combination of two or more of the embodiments can be used, as desired.

A flow line 108 can, if desired, lead from the flow line 42 to the flow line 96 upstream of the product withdrawal stage 98, the drying and granulation stage 100, and/or the citric acid recovery unit 102.

In use, clarified citric acid fermentation broth, produced in known fashion in the fermentation stage, passes to the cation exchanger 32 where it is contacted with a suitable resin to remove cations such as calcium and sodium ions. If these ions are not removed they would form complexes with the citrate ions and be retained by the nanofilter element in the subsequent filtration stage 40 leading to product losses. The resin bed can be regenerated in known fashion, when required.

The broth then passes to the nanofiltration stage 40 where the citric acid is separated, by contacting the broth with a nanofiltration membrane, from glucose, fructose, and higher molecular weight components in the broth such as protein, residual anti-foaming agents, sucrose, peptides and polysaccharides which thus form the retentate. Smaller molecules as well as some anions pass through the nanofiltration membrane and, together with the citric acid and most of the water, form the permeate. The permeate is thus in the form of a purified citric acid solution in which the ratio or proportion of the concentration of citric acid to that of glucose and fructose is greater than the ratio or proportion of the concentration of citric acid to that of the glucose and fructose in the feed to the stage 40. Thus, in the filtration stage 40, glucose and fructose, which have a similar molecular weight (180) to citric acid (192) are separated therefrom.

The permeate from the filtration stage 40 passes to the anionic exchanger 46 where anionic impurities are removed and withdrawn. The resin bed of the anionic exchanger 46 is regenerated in known fashion, when required.

The citric acid containing solution from the exchanger 46 passes to the activated carbon bed stage 52 where traces of organics are removed.

The citric acid solution thereafter passes to the evaporator where it is concentrated, using steam, from a concentration of 15% to 20% by mass citric acid, typically up to about 65% to 80% by mass citric acid. Condensate from the evaporation stage 56 leaves along the line 60. The concentrated citric acid solution passes to the first crystallization stage 64 where crystallization of the citric acid is effected. The stream then passes to the first centrifuge stage 68 where the citric acid crystals are separated from the mother liquor. The citric acid crystals pass into the dissolution tank 72 where they are redissolved in make-up water, whereafter they are recrystallized in the second crystallization stage 78 to improve crystal quality. The make-up water may be obtained from any suitable source, such as process condensate, a dilute citric acid stream, or the like. The stream from the crystallization stage 78 passes to the second centrifugation stage 82 where mother liquor is again removed. The moist crystals pass to the drier 88, with dried crystals passing to the screening stage 92. Dried solid substantially pure citric acid crystals are withdrawn along the flow line 94.

The crystallization stages 64, 78 typically comprise known crystallizers, and will thus include ancillary equipment normally associated therewith such as steam feed/condensate outlet lines, cooling fluid lines, and the like.

Mother liquor from the first centrifugation stage 68 is withdrawn along the flow line 96.

In a first embodiment, a portion of this mother liquor can be recycled to the activated carbon bed 52.

In a second embodiment, at least a portion of this mother liquor can be withdrawn as a liquid product in the stage 98.

In a third embodiment, at least a portion of this mother liquor can be dried and granulated in the stage 100 to obtain a citric acid/carbohydrate solid commercial product.

In a fourth embodiment, at least a portion of this mother liquor can pass to the recovery stage 102. Waste product, e.g., glucose and trace impurities, from the recovery stage 102 is withdrawn, while if pure citric acid is recovered, it may be recycled to upstream or downstream of stage 40; or if citrate salts are recovered, they will be recovered as product. A portion of the retentate from the nanofiltration stage 40 can be routed, by means of the flow line 108, to the stream 96 and then routed to any of the optional stages 98, 100 and/or 102, if desired, to recover residual citric acid or a derivative thereof present in this stream.

In one version of the invention, the recovery stage 102 may utilize calcium citrate precipitation after lime addition; followed by sulphuric acid addition to form citric acid as well as the by product gypsum, to recover citric acid.

In another version, the citric acid in the mother liquor may, in the stage 102, be extracted using a suitable solvent, followed by re-extraction of citric acid from the solvent phase into water using concentration differences or with the aid of heat.

In yet another version, the recovery stage 102 may comprise an ion exchange resin which selectively adsorbs citric acid, with elution of the product into water thereafter taking place.

In yet a further version of the invention, the citric acid recovery stage may comprise various types of chromatography.

The Applicant believes that with the process 10, citric acid can be recovered effectively and at relatively low cost. In addition, it is believed that the process 10 will be relatively simple to operate.

### Example 1

The process 10 of the invention was simulated theoretically as follows:

Clarified citric acid fermentation broth containing 18.4 weight percent citric acid, can be obtained by fermentating various cultures, such as *Aspergillus niger,* on a purified carbohydrate feedstock, and filtering off the resultant biomass. The broth leaving the fermenters can contain 0.2% (w/w) unfermented glucose or 0.2% (w/w) unfermented fructose.

The clarified citric acid fermentation broth is then subjected to cation exchange, to remove cations such as potassium and magnesium ions.

The clarified decationized citric acid fermentation broth is then contacted with a nanofiltration membrane, and 80 or more percent of the citric acid transfers to the permeate, which contains up to 18 weight percent citric acid. The permeate also contains the following from the clarified decationized citric acid fermentation broth: a portion of the glucose and fructose, anions, cations, amino acids and sucrose, as well as 80 or more percent of the water. The retentate can be treated in a citric acid recovery step, using the UOP™ Citric Acid Sorbex™ Process (Citrex™), to recover the remaining citric acid.

The permeate from the nanofiltration step can be subjected to anion exchange, to remove traces of anionic impurities, followed by contacting with activated carbon, to remove traces of organics.

The permeate can thereafter be concentrated by evaporation in an evaporator, followed by a first crystallizer, a first centrifuge, a dissolution tank, a second crystallizer and a second centrifuge; with 20% by weight of the mother liquor from the second centrifuge being recycled to the first crystallizer, while the mother liquor from the first centrifuge is withdrawn.

The process can include (i) recycling 25% (w/w) of the mother liquor from the first centrifuge to upstream of the evaporator, (ii) withdrawing 10.0% (w/w) of the mother liquor from the first centrifuge as a liquid product, (iii) drying and granulating 21.6% (w/w) of the mother liquor from the first centrifuge to obtain a solid citric acid/carbohydrate product; and (iv) treating the remainder of the mother liquor from the first centrifuge, together with 80% (w/w) of the nanofiltration retentate, using the UOP™ Citric Acid Sorbex™ Process (Citrex™) process (this process revolves around any one of various chromatographic techniques, such as ion exclusion chromatography, whereby citric acid is separated from the feed stream by selective adsorption onto a solid adsorbent) in a recovery step, to recover citric acid which can be recycled to downstream of the nanofiltration step.

In the Citrex recovery step, which uses a very dilute solution of sulfuric acid as desorbent, the extract can contain, from the feed stream, on a weight to weight basis: 92% of the citric acid, 1% of the glucose and fructose, 1% of the cations and anions, 1% of the amino acids and biomass, negligible sulfuric acid, and 44% of the water from both the feed stream and the desorbent stream. The balance of the above mentioned components report to the raffinate (waste stream).

### Example 2

In a simulation of the nanofiltration step or stage 40, laboratory scale tests were conducted on simulated citric acid fermentation broths containing, by mass, 18-19% citric acid, 1% lactose, 0.2% glucose and 0.05% yeast extract. The yeast extract was used to mimic other components normally present in commercial formation broths. Each test was conducted with a pair of membranes, by treating a batch of the simulated broth. Concentrations of each of the components were measured, and the rejections calculated. The results are set out in Tables 1, 2 and 3 (all percentages are on a mass bases).

**TABLE 1 -**

| Results of Nanofiltration Test 1 | | | |
|---|---|---|---|
| Experiment 1 | Citric Acid % | Lactose % | Glucose % |
| Feed | 18.8 | 0.88 | 0.22 |
| Permeate - Membrane A | 12.3 | 0.01 | 0.03 |
| Permeate - Membrane B | 14.6 | 0.18 | 0.09 |
| Concentrate | 29 | 2.1 | 0.47 |
| Membrane A: Filmtec NF45 membrane obtained from Dow Liquid Separations in the USA or from Dow Deutschland Inc., Industriestrasse, 77836 Rheinmunster, Germany. Membrane B: MPKW MPF21 membrane obtained from Membrane Products Kiryate Weizman Limited, Post Office Box 138, Rehovot 76101, Israel. | | | |

**TABLE 2 -**

| Results of Nanofiltration Test 2 | | | |
|---|---|---|---|
| Experiment 2 | Citric acid % | Lactose % | Glucose % |
| Feed | 18 | 0.88 | 0.20 |
| Permeate - Membrane A | 11.4 | 0.01 | none |
| Permeate - Membrane B | 11.7 | 0.05 | 0.07 |
| Concentrate | 28 | 1.9 | 0.38 |

**TABLE 3 -**

| Rejections of the two membranes | | | |
|---|---|---|---|
| Rejections expressed as percentages | Citric acid % | Lactose % | Glucose % |
| Filmtec NF45 Test 1 | 34.6 | 98.9 | 86.4 |
| Test 2 | 36.7 | 98.9 | >90 |
| MPKW MPF23 Test 1 | 22.3 | 79.5 | 59.1 |
| Test 2 | 35.0 | 94.3 | 65.0 |

One of the key parameters in nanofiltration is the rejection. For the simulated citric acid fermentation broths, it was expected, according to literature and product information, that membrane rejections would be in the order lactose > citric acid > glucose. However, as can be seen from Table 3, the actual rejection of citric acid was surprisingly found to be lower than that of glucose.

This feature thus provides the basis for a simple and efficient means of separating citric acid from glucose and/or fructose, especially removing most of the residual glucose, in respect of fermentation broth.

## Claims

1. A process for purifying citric acid, which process comprises
subjecting a liquid containing, in solution, citric acid as well as glucose and/or fructose to nanofiltration in a filtration step;
obtaining, from the filtration step, a permeate in which the ratio of the concentration of the citric acid to that of glucose and/or fructose is greater than the ratio of the concentration of the citric acid to that of glucose and/or fructose in the solution;
**characterized in that**
the rejection rate of citric acid on the nanofiltration membrane is lower than the rejection rate of glucose and/or fructose and that the permeate is concentrated by subjecting it to crystallization without treating the permeate to remove the remaining glucose and/or fructose prior to the crystallization.

2. The process according to claim 1, wherein the liquid is clarified citric acid fermentation broth so that the permeate is a purified citric acid solution and the liquid includes glucose.

3. The process according to claim 2, wherein the filtration step is carried out at a concentration of the citric acid in the broth of 5%-30% by mass, and wherein the filtration is carried out at a temperature of 10°C-100°C.

4. The process according to claim 2 or claim 3, wherein the clarified citric acid fermentation broth is, before the filtration step, subjected to cation exchange to remove cations therefrom.

5. The process according to any one of claims 2 to 4, wherein the citric acid solution from the filtration step is purified by anion exchange and/or by contacting it with activated carbon.

6. The process according to any one of claims 1 to 5, wherein the concentration is effected by passing the purified citric acid solution sequentially through an evaporator; a first crystallizer; a first centrifuge; optionally, a dissolution tank, a second crystallizer and a second centrifuge; and producing mother liquor in the first centrifuge and, when present, in the second centrifuge, with a portion of the mother liquor from the second centrifuge then being recycled to the first crystallizer, while the mother liquor from the first centrifuge is withdrawn.

7. A process according to claim 6, which includes (i) recycling a portion of the mother liquor from the first centrifuge to upstream of the evaporator; and/or (ii) withdrawing at least a portion of the mother liquor from the first centrifuge as a liquid product; and/or (iii) drying and granulating at least a portion of the mother liquor from the first centrifuge to obtain a solid citric acid/carbohydrate product and/or (iv) treating at least a portion of the mother liquor from the first centrifuge, in a recovery step, to recover citric acid to recycle, or citrate salts as product.

8. The process according to claim 6, which includes treating at least a portion of the mother liquid from the first centrifuge, in a recovery step, to recover citric acid, with the citric acid being recycled to upstream and/or downstream of the nanofiltration step, and with treatment in the recovery step comprising one of the following: calcium citrate precipitation by adding lime thereto and redissolving with sulphuric acid; solvent extraction of citric acid utilizing a suitable solvent, followed by re-extraction of citric acid from the solvent into water using concentration differences or heating; ion exchange using a resin which selectively adsorbs citric acid, followed by elution; or chromatography.

9. The process according to any one of claims 1 to 8, which includes (i) withdrawing at least a portion of the retentate from the filtration step as a liquid product, and/or (ii) drying or granulating at least a portion of the retentate from the filtration step to obtain a solid citric acid product, and/or (iii) treating at least a portion of the retentate from the filtration step in a citric acid recovery step.

10. The process according to claim 1, wherein
the crystallization is conducted by concentrating the permeate by subjecting it to crystallization in at least one crystallizer, with citric acid crystals and mother liquor being produced in the crystallizer;
separating the citric acid crystals from the mother liquor;
treating at least a portion of the mother liquor to recover citric acid therefrom; and
recycling at least a portion of the recovered citric acid to upstream and/or downstream of the filtration step.

11. The process according to claim 10, wherein the liquid is clarified citric acid fermentation broth so that the permeate is a purified citric acid solution and the liquid includes glucose.

12. The process according to claim 10 or 11, wherein recovery of the citric acid is effected by means of chromatography.

## Patentansprüche

1. Verfahren zur Reinigung von Citronensäure, wobei das Verfahren folgendes umfasst:
Unterziehen einer Flüssigkeit, die, in Lösung Citronensäure sowie Glucose und/oder Fructose enthält, einer Nanofiltration in einem Filtrationsschritt,
Erhalten eines Permeats aus dem Filtrationsschritt, in dem das Verhältnis der Konzentration der Citronensäure zu der von Glucose und/oder Fructose größer als das Verhältnis der Konzentration der Citronensäure zu der von Glucose und/oder Fructose in der Lösung ist,
**dadurch gekennzeichnet, dass**
die Entfernungsrate von Citronensäure niedriger als die Entfernungsrate von Glucose und/oder Fructose ist und dass das Permeat durch sein Unterziehen einer Kristallisation konzentriert wird, ohne das Permeat zu behandeln, um die verbleibende Glucose und/oder Fructose vor der Kristallisation zu entfernen.

2. Das Verfahren nach Anspruch 1, wobei die Flüssigkeit geklärte Citronensäure-Fermentationsbrühe ist, so dass das Permeat eine gereinigte Citronensäurelösung ist und die Flüssigkeit Glucose einschließt.

3. Das Verfahren nach Anspruch 2, wobei der Filtrationsschritt bei einer Konzentration der Citronensäure in der Brühe von 5 - 30 Massenprozente durchgeführt wird und wobei die Filtration bei einer Temperatur von 10°C - 100°C durchgeführt wird.

4. Das Verfahren nach Anspruch 2 oder Anspruch 3, wobei die geklärte Citronensäure-Fermentationsbrühe vor der Filtrationsstufe einem Kationenaustausch unterzogen wird, um die Kationen daraus zu entfernen.

5. Das Verfahren nach irgendeinen der Ansprüche 2 bis 4, wobei die Citronensäurelösung des Filtrationsschritts durch Anionenaustausch und/oder durch in Kontakt-Bringen mit Aktivkohle gereinigt wird.

6. Das Verfahren nach irgendeinen der Ansprüche 1 bis 5, wobei die Konzentrierung durch das Durchlaufen der gereinigten Citronensäurelösung aufeinanderfolgend durch einen Verdampfer, eine erste Kristallisationsvorrichtung eine erste Zentrifuge, wahlweise ein Auflösungsbehälter, eine zweite Kristallisationsvorrichtung und eine zweite Zentrifuge ausgeführt wird, und Herstellung von Mutterlauge in der ersten Zentrifuge und, wenn vorhanden, in der zweiten Zentrifuge, wobei ein Teil der Mutterlauge der zweiten Zentrifuge dann zu der ersten Kristallisationsvorrichtung zurückgeführt wird, während die Mutterlauge der ersten Zentrifuge abgeleitet wird.

7. Verfahren nach Anspruch 6, welches einschließt (i) Rückführen eines Teils der Mutterlauge der ersten Zentrifuge zu einem Punkt stromaufwärts vom Verdampfer; und/oder (ii) Ableiten von wenigstens einem Teil der Mutterlauge der ersten Zentrifuge als ein flüssiges Produkt; und/oder (iii) Trocknen und/oder Granulieren von wenigstens einem Teil der Mutterlauge der ersten Zentrifuge, um ein festes Citronensäure/Kohlenhydrat-Produkt zu erhalten; und/oder (iv) Behandeln von wenigstens einem Teil der Mutterlauge der ersten Zentrifuge in einem Wiedergewinnungsschritt, um die Citronensäure für die Wiederverwertung oder Citratsalze als Produkte wiederzugewinnen.

8. Verfahren nach Anspruch 6, das die Behandlung von wenigstens einem Teil der Mutterlauge der ersten Zentrifuge in dem Wiedergewinnungsschritt für die Wiedergewinnung von Citronensäure einschließt, indem die Citronensäure zu einem Punkt stromaufwärts oder abwärts des Nanofiltrationsschritts zurückgeführt wird, und wobei die Behandlung in dem Wiedergewinnungsschritt eines des folgenden umfasst: Calciumcitratausfällung durch Zusatz von Kalk dazu und Wiederauflösung mit Schwefelsäure, Lösungsmittelextraktion unter Verwendung eines geeigneten Lösungsmittels, gefolgt von erneuter Extraktion der Citronensäure von dem Lösungsmittel in Wasser unter Verwendung von Konzentrationsunterschieden oder Erwärmen, Ionenaustausch unter Verwendung eines selektiv Citronensäure adsorbierenden Harzes, gefolgt von Elution oder Chromatographie.

9. Das Verfahren nach irgendeinen der Ansprüche 1 bis 8, das beinhaltet: (i) Ableiten wenigstens einen Teil des Retentats des Filtrationsschritts als ein flüssiges Produkt und/oder (ii) Trocknen oder Granulieren von wenigstens einem Teil des Retentats des Filtrationsschritts, um ein festes Citronensäureprodukt zu erhalten; und/oder (iii) Behandeln von wenigstens einem Teil des Retentats des Filtrationsschritts in einem Citronensäurewiedergewinnungsschritt.

10. Das Verfahren nach Anspruch 1, wobei
die Kristallisation durch Konzentrieren und Unterziehen einer Kristallisation des Permeats in wenigstens einer Kristallisationsvorrichtung durchgeführt wird, wobei die Citronensäurekristalle und Mutterlauge in der Kristallisationsvorrichtung erzeugt werden;
Trennen der Citronensäurekristalle von der Mutterlauge;
Behandeln von wenigstens einem Teil der Mutterlauge, um Citronensäure daraus zurückzugewinnen; und
Rückführen von wenigstens einem Teil der wiedergewonnenen Citronensäure zu einem Punkt stromaufwärts und/oder abwärts des Filtrationsschritts.

11. Das Verfahren nach Anspruch 10, wobei die Flüssigkeit geklärte Citronensäurefermentationsbrühe ist, so dass das Permeat eine gereinigte Citronensäurelösung ist und die Flüssigkeit Glucose einschließt.

12. Das Verfahren nach Anspruch 10 oder 11, wobei die Wiedergewinnung der Citronensäure mittels Chromatographie durchgeführt wird.

## Revendications

1. Procédé pour purifier de l'acide citrique, lequel procédé comprend les étapes consistant :
à soumettre un liquide contenant, en solution, de l'acide citrique ainsi que du glucose et/ou du fructose, à une nanofiltration dans une étape de filtration ;
à obtenir, à partir de l'étape de filtration, un perméat dans lequel le rapport de la concentration de l'acide nitrique à celle du glucose et/ou du fructose est plus grande que le rapport de la concentration de l'acide nitrique à celle du glucose et/ou du fructose dans la solution ;
**caractérisé en ce que**
le taux de réjection de l'acide nitrique sur la membrane de nanofiltration est plus faible que le taux de réjection du glucose et/ou du fructose, et **en ce que** le perméat est concentré en le soumettant à une cristallisation, sans traiter le perméat pour enlever le glucose et/ou le fructose restant avant la cristallisation.

2. Procédé selon la revendication 1, dans lequel le liquide est un bouillon de fermentation contenant de l'acide citrique clarifié, de sorte que le perméat est une solution d'acide citrique purifiée, et le liquide inclus du glucose.

3. Procédé selon la revendication 2, dans lequel l'étape de filtration est effectuée à une concentration de 5% à 30% en poids de l'acide citrique dans le bouillon, et dans lequel la filtration est effectuée à une température de 10°C à 100°C.

4. Procédé selon la revendication 2 ou 3, dans lequel le bouillon de fermentation contenant de l'acide citrique clarifié est soumis, avant l'étape de filtration, à un échange de cations pour en enlever des cations.

5. Procédé selon l'une quelconque des revendications 2 à 4, dans lequel la solution d'acide citrique provenant de l'étape de filtration est purifiée par échange d'anions et/ou en la mettant en contact avec du charbon actif.

6. Procédé selon l'une quelconque des revendications 1 à 5, dans laquelle on effectue la concentration en faisant passer la solution d'acide citrique purifiée séquentiellement à travers un évaporateur ; un premier cristalliseur ; une première centrifugeuse ; optionnellement un réservoir de dissolution, un second cristalliseur et une seconde centrifugeuse ; et en produisant une liqueur mère dans la première centrifugeuse et, lorsqu'elle est présente, dans la seconde centrifugeuse, une partie de la liqueur mère provenant de la seconde centrifugeuse étant ensuite recyclée dans la première centrifugeuse, tandis que la liqueur mère provenant de la première centrifugeuse est soutirée.

7. Procédé selon la revendication 6, qui comporte (i) un recyclage d'une partie de la liqueur mère provenant de la première centrifugeuse vers l'amont de l'évaporateur ; et/ou (ii) un soutirage d'au moins une partie de la liqueur mère provenant de la première centrifugeuse en tant que produit liquide ; et/ou (iii) un séchage et une granulation d'au moins une partie de la liqueur mère provenant de la première centrifugeuse afin d'obtenir un produit solide d'acide citrique et de glucide ; et/ou (iv) un traitement d'au moins une partie de la liqueur mère provenant de la première centrifugeuse, dans une étape de récupération, afin de récupérer de l'acide citrique à recycler ou des sels de citrate en tant que produit.

8. Procédé selon la revendication 6, qui comporte un traitement d'au moins une partie de la liqueur mère provenant de la première centrifugeuse, dans une étape de récupération, afin de récupérer de l'acide citrique, l'acide citrique étant recyclé vers l'amont et/ou vers l'aval de l'étape de nanofiltration, et le traitement dans l'étape de récupération comprenant une des opérations suivantes : une précipitation de citrate de calcium par addition de chaux et redissolution avec de l'acide sulfurique ; une extraction par solvant de l'acide citrique en utilisant un solvant convenable, suivie d'une réextraction de l'acide sulfurique à partir du solvant dans de l'eau en utilisant des différences de concentration ou un chauffage ; un échange d'ions en utilisant une résine qui absorbe sélectivement l'acide citrique, suivie d'une élution ; ou une chromatographie.

9. Procédé selon l'une quelconque des revendications 1 à 8, qui comporte (i) un soutirage d'au moins une partie du rétentat provenant de l'étape de filtration en tant que produit liquide, et/ou (ii) un séchage ou une granulation d'au moins une partie du rétentat provenant de l'étape de filtration afin d'obtenir un produit solide d'acide citrique, et/ou (iii) un traitement d'au moins une partie du rétentat provenant de l'étape de filtration dans une étape de récupération de l'acide citrique.

10. Procédé selon la revendication 1, dans lequel
la cristallisation est conduite en concentrant le perméat en le soumettant à une cristallisation dans au moins un cristalliseur, des cristaux d'acide citrique et une liqueur mère étant produits dans le cristalliseur ;
on sépare les cristaux d'acide citrique de la liqueur mère ;
on traite au moins une partie de la liqueur mère afin d'en récupérer l'acide citrique ; et
on recycle au moins une partie de l'acide citrique récupéré vers l'amont et/ou vers l'aval de l'étape de filtration.

11. Procédé selon la revendication 10, dans lequel le liquide est un bouillon de fermentation contenant de l'acide citrique clarifié, de sorte que le perméat est une solution d'acide citrique purifiée, et le liquide contient du glucose.

12. Procédé selon la revendication 10 ou 11, dans lequel la récupération de l'acide citrique est effectuée au moyen d'une chromatographie.
